# EUROPÄISCHE PATENTSCHRIFT

(11) **EP 3 588 122 B1**
(45) Veröffentlichungstag und Bekanntmachung des Hinweises auf die Patenterteilung: **16.03.2022**
(21) Anmeldenummer: 18180414.7
(22) Anmeldetag: 28.06.2018
(51) Int. Cl.: G01R 33/561, G01R 33/565, G01R 33/483, G01R 33/567

(54) **VERFAHREN ZUR AUFNAHME VON MAGNETRESONANZDATEN UMFASSEND DIE AUFNAHME VON FETTSELEKTIVEN NAVIGATORDATEN MITTELS SIMULTANER MEHRSCHICHTBILDGEBUNG**
METHOD FOR RECORDING MAGNETIC RESONANCE DATA COMPRISING THE ACQUISITION OF FAT NAVIGATORS USING SIMULTANEOUS MULTI-SLICE ACQUISITION
PROCÉDÉ D'ENREGISTREMENT DE DONNÉES DE RÉSONANCE MAGNÉTIQUE COMPRENANT UNE ACQUISITION DES NAVIGATEURS POUR LA GRAISSE MULTI-TRANCHE SIMULTANÉE

(43) Veröffentlichungstag der Anmeldung: 01.01.2020
(73) Patentinhaber: Siemens Healthcare GmbH, 91052 Erlangen (DE)
(72) Erfinder: Zeller, Mario, 91054 Erlangen (DE)

(56) Entgegenhaltungen:
- EP-A1- 3 118 644
- DE-A1-102015 222 835
- DANIEL GALLICHAN ET AL: "Retrospective correction of involuntary microscopic head movement using highly accelerated fat image navigators (3D FatNavs) at 7T", MAGNETIC RESONANCE IN MEDICINE., Bd. 75, Nr. 3, 14. April 2015 (2015-04-14) , Seiten 1030-1039, XP055539650, US ISSN: 0740-3194, DOI: 10.1002/mrm.25670
- KAWIN SETSOMPOP ET AL: "Blipped-controlled aliasing in parallel imaging for simultaneous multislice echo planar imaging with reduced g-factor penalty", MAGNETIC RESONANCE IN MEDICINE, Bd. 67, Nr. 5, 1. Mai 2012 (2012-05-01), Seiten 1210-1224, XP055027257, ISSN: 0740-3194, DOI: 10.1002/mrm.23097

## Beschreibung

Die Erfindung betrifft ein Verfahren zur Aufnahme von Magnetresonanzdaten eines Aufnahmebereichs eines Patienten wie in Anspruch 1 definiert. Daneben betrifft die Erfindung eine Magnetresonanzeinrichtung, ein Computerprogramm und einen elektronisch lesbaren Datenträger.

Die Magnetresonanzbildgebung stellt ein inzwischen gängiges Bildaufnahmemittel insbesondere bei der medizinischen Bildgebung an Patienten dar. Dabei ist bekannt, dass viele Magnetresonanzsequenzen bzw. im Allgemeinen Aufnahmevorgänge zum Erhalt einer möglichst guten diagnostischen Bildqualität eine längere Zeit in Anspruch nehmen. Während dieser Zeit können Bewegungen des Patienten nicht gänzlich ausgeschlossen werden, so dass die sogenannten Bewegungsartefakte ein wichtiges Thema bei der modernen Magnetresonanzbildgebung darstellen. Auftretende Bewegung während der Aufnahme von Magnetresonanzdaten eines Aufnahmebereichs eines Patienten kann die Bildqualität, mithin auch die diagnostische Eignung, stark einschränken und in Extremfällen sogar eine Neuaufnahme erfordern.

Im Stand der Technik wurde bereits eine Vielzahl von Ansätzen zur Vermeidung von Bewegungen und/oder zur Bewegungskorrektur vorgeschlagen. Grundsätzlich bekannte Herangehensweisen umfassen neben sequenzinhärenten Navigatoren auch externe Sensoren wie Kameras, Magnetfeldmessungen oder sogenannte "pilot tone"-Aufnahmen. Dabei sind sowohl prospektive wie auch retrospektive Korrekturansätze bekannt. Es existieren ferner im Stand der Technik bereits Verfahren, bei denen Magnetresonanzdaten durch bestimmte Atem- und/oder EKG-Zustände getriggert werden. Auch hier kann die Festlegung der Aufnahmezeitpunkte durch sogenannte Navigatoren erfolgen, es ist jedoch auch eine Verwendung von externen Sensoren denkbar. Navigatoren sind dabei Magnetresonanzaufnahmen, die nur eine äußerst kurze Zeit in Anspruch nehmen und zumindest ein Merkmal des Patienten hinreichend deutlich zeigen, um Schlussfolgerungen auf die Bewegung zu ziehen. Beispielsweise wurden bereits eindimensionale Navigatoren, in denen die Position des Zwerchfells erkennbar ist, zur Nachverfolgung der Atmung und/oder zur Atemtriggerung vorgeschlagen.

Weitere Ansätze, um Bewegungsartefakte in Magnetresonanzdaten zu reduzieren, umfassen die Verwendung von Magnetresonanzsequenzen, die inhärent robust gegenüber Bewegungen sind, beispielsweise radiale Magnetresonanzsequenzen und BLADE-Magnetresonanzsequenzen, bei denen eine möglichst kurze Messzeit gegeben ist, um die Wahrscheinlichkeit von Bewegungen zu reduzieren, oder die mehrere Mittelungen umfassen, um Bewegungseinflüsse auszumitteln.

Heutige vereinfachte Benutzeroberflächen von Magnetresonanzeinrichtungen bieten dem Benutzer hinsichtlich von Bewegungsartefakten meist Gesamtstrategien an, beispielsweise "hochauflösend", "schnell" und/oder "bewegungsrobust", wobei "bewegungsrobuste" Strategien hierbei häufig auf automatische Weise oben genannte Ansätze zur Verringerung von Bewegungsartefakten kombinieren, wobei allerdings häufig Nachteile hinsichtlich der Auflösung der resultierenden Magnetresonanzdaten und/oder der Geschwindigkeit der Gesamtaufnahme vorliegen.

In den letzten Jahren haben sogenannte Fettnavigatoren als sequenzinhärente Navigatoren, das bedeutet, zwischen einzelnen Aufnahmesegmenten bzw. Aufnahmezeitabschnitten ausgespielte Navigatoren, an Bedeutung gewonnen. Dies gilt insbesondere für die Aufnahme von Magnetresonanzdaten im Kopfbereich des Patienten, da dort das seltene Vorkommen von Fett (räumliche Spärlichkeit - "sparsity") gewährleistet ist, da Fett im Wesentlichen nur in den Randbereichen der Anatomie auftritt. Im Stand der Technik bekannte Fettnavigatoren nutzen eine Fettnavigatorsequenz, die in einem Anregungsmodul mittels eines oder mehrerer Hochfrequenzpulse in einem zweidimensionalen oder dreidimensionalen Aufnahmebereich, insbesondere einer Schicht oder einem dicken Slab, Spins fettselektiv anregt, das bedeutet, Signale von wasseräquivalenten Spins werden unterdrückt. Hierfür können beispielsweise Binomialpulsfolgen im Anregungsmodul verwendet werden.

In einem Artikel von Mathias Engström et al., "Collapsed fat navigators for brain 3D rigid body motion", Magnetic Resonance Imaging 33 (2015) 984 - 991, wird beispielsweise vorgeschlagen, eine Fettnavigatorsequenz ("collapsed FatNav") einer diffusionsgewichteten 3D-Mehrfach-Slab-EPI-Sequenz hinzuzufügen. Hierbei werden drei orthogonale EPI-Auslesemodule verwendet, um starre Bewegungen des Kopfes im Bildraum nachzuvollziehen und prospektive Bewegungskorrekturen durchzuführen. Konkret wird dort vorgeschlagen, das Signal des führenden Fettsättigungs-Hochfrequenzpulses der Diffusionssequenz auch für den Fettnavigator zu nutzen, so dass lediglich das Auslesemodul benötigt wird und die Gesamtaufnahmezeit nur unwesentlich erhöht wird. Derartige Fettnavigatoren lassen sich jedoch auch außerhalb der Diffusionsbildgebung einsetzen und nutzen, wie bereits durch Engström et al. beschrieben, diverse Unterabtastungsschemata, wodurch letztlich die beschriebene "sparsity" von Fett in verschiedenen Aufnahmebereichen, insbesondere im Bereich des Kopfes eines Patienten, ausgenutzt wird.

Bei zweidimensionalen Fettnavigatoren werden dabei üblicherweise mehrere Schichten innerhalb des für den Fettnavigator bereitgestellten Navigatorzeitabschnitts aufgenommen, um Bewegungen in allen drei Raumdimensionen nachvollziehen zu können. Daher sind bei zweidimensionalen Fettnavigatoren des Standes der Technik nur geringe in-plane-Auflösungen erzielbar. Dreidimensionale Fettnavigatoren haben den Nachteil, dass in drei orthogonalen Richtungen aufgenommen werden soll und es innerhalb des zur Verfügung stehenden Navigatorzeitabschnitts schwierig wird, insbesondere in der k_{z}-Richtung eine hinreichend hohe räumliche Auflösung zu erzielen. Insbesondere sind die sogenannten "slabs" der dreidimensionalen Aufnahmen in z-Richtung deutlich dicker und häufig auch schlechter aufgelöst als Schichten eines Schichtstapels bei zweidimensionalen Aufnahmevarianten.

In der Patentschrift DE102015222835 ist ein Verfahren zur Aufnahme von Magnetresonanzdaten offenbart, wobei jeweils zwei Navigatorschichten und eine Bildgebungsschicht gleichzeitig akquiriert werden. In Daniel Gallichan et al., Retrospective correction of involuntary microscopic head movement using highly accelerated fat image navigators (3D FatNavs) at 7T, Magnetic Resonance in Medicine 2016; 75(3): 1030-1039 ist ein Verfahren zur Bewegungskorrektur von Magnetresonanzdaten mittels 3D-kodierten Fettnavigatordaten offenbart. In der Patentschrift EP3118644 ist ein Verfahren zur Beschleunigung von herkömmlichen Navigatoraufnahmen mittels simultaner Mehrschichtbildgebung offenbart.

Der Erfindung liegt daher die Aufgabe zugrunde, einen Fettnavigator für die Aufnahme von Magnetresonanzdaten bereitzustellen, der eine hinreichend feine Schichtauflösung besitzt und dennoch die Navigatordaten in möglichst kurzer Zeit akquirieren kann. Zur Lösung dieser Aufgabe sind erfindungsgemäß ein Verfahren, eine Magnetresonanzeinrichtung, ein Computerprogramm und ein elektronisch lesbarer Datenträger mit den Merkmalen der unabhängigen Ansprüche vorgesehen. Vorteilhafte Ausgestaltungen ergeben sich aus den Unteransprüchen.

Bei einem Verfahren der eingangs genannten Art ist mithin erfindungsgemäß vorgesehen, dass die Navigatordaten mittels der simultane Mehrschichtbildgebung nutzenden Fettnavigatorsequenz nach dem auf mehrere aufzunehmende Fettnavigatorschichten wirkenden Anregungsmodul im Auslesemodul simultan aus den angeregten Fettnavigatorschichten aufgenommen werden.

Es wird mithin vorgeschlagen, für den Fettnavigator die sogenannte simultane Mehrschichtbildgebung (Simultaneous MultiSlice - SMS) einzusetzen. Dabei werden durch ein gemeinsames

Anregungsmodul mehrere Schichten innerhalb einer Repetition angeregt und die Magnetresonanzsignale aus diesen Schichten werden gleichzeitig vermessen. Anschließend werden Navigatordaten der einzelnen Fettnavigatorschichten algorithmisch voneinander getrennt, beispielsweise unter Nutzung von Verfahren wie GRAPPA. Bekannte SMS-Vorgehensweisen umfassen beispielsweise die sogenannte Hadamard-Kodierung, Verfahren mit simultaner Echo-Refokussierung, Verfahren mit Breitband-Datenaufnahme oder auch Verfahren, die eine parallele Bildgebung in Schichtrichtung einsetzen. Zu den letztgenannten Verfahren gehört beispielsweise auch die Blipped-CAIPI-Technik, wie sie von Setsompop et al. in "Blipped-Controlled Aliasing in Parallel Imaging for Simultaneous Multislice Echo Planar Imaging With Reduced g-Factor Penalty", Magnetic Resonance in Medicine 67, 2012, Seiten 1210 - 1224, beschrieben wird.

Bei derartigen Schichtmultiplexing-Verfahren wird im Anregungsmodul ein sogenannter Multiband-Hochfrequenzpuls verwendet, um zwei oder mehr Schichten gleichzeitig anzuregen oder anderweitig zu manipulieren, beispielsweise zu refokussieren oder, wie im vorliegenden Fall des Fettnavigators, selektiv zu sättigen/anzuregen. Beispielsweise kann ein solcher Multiband-Hochfrequenzpuls ein Multiplex von individuellen Einzelschicht-Hochfrequenzpulsen sein, die zur Manipulation der einzelnen gleichzeitig zu manipulierenden Schichten verwendet werden. Um die resultierenden Magnetresonanzsignale der verschiedenen Fettnavigatorschichten trennen zu können, wird beispielsweise den individuellen Hochfrequenzpulsen vor dem Multiplexing, insbesondere durch Addieren eines linearen Phasenanstiegs, je eine unterschiedliche Phase aufgeprägt, wodurch die Schichten im Ortsraum gegeneinander verschoben werden. Durch das Multiplexing erhält man beispielsweise einen Basisband-modulierten Multiband-Hochfrequenzpuls durch Summieren der Pulsformen der individuellen Einzelschicht-Hochfrequenzpulse.

Die Anwendung der simultanen Mehrschichtbildgebung für Fettnavigatoren bringt eine Vielzahl von Vorteilen mit sich. Aufgrund der bereits erwähnten hohen "sparsity" von Fett, insbesondere bei Aufnahmen im Bereich des Kopfes eines Patienten, ist dabei ein besonders hoher Multibandfaktor, das bedeutet, viele zeitgleich aufgenommene Fettnavigatorschichten, möglich, wobei bevorzugt wenigstens drei, insbesondere wenigstens acht, Fettnavigatorschichten simultan aufgenommen werden können. Insbesondere in Kombination mit einer in-plane-Unterabtastung, wie beschrieben, lassen sich hierdurch äußerst schnelle Aufnahmen der Navigatordaten erreichen. Dabei kann der in-plane-Unterabtastungsfaktor mehr als zwei, insbesondere mehr als zehn, betragen. Bei der Herleitung der Navigatordaten aus den aufgenommenen Magnetresonanzsignalen wird dann zunächst ein sogenannter Schicht-GRAPPA-Algorithmus zur Trennung der Schichten angewandt, wonach ein in-plane-GRAPPA-Algorithmus genutzt werden kann, um dort Aliasing-Effekte aufzulösen. Alternativ kann auch ein Algorithmus zum Einsatz kommen, welcher Schichttrennung und in-plane-Rekonstruktion in einem Schritt durchführt.

Im Vergleich zu konventionellen dreidimensionalen Fettnavigatoren erfolgt das Anregen und das Auslesen der Fettnavigatorschichten vorliegend tatsächlich zeitgleich, mithin simultan. Dies erlaubt eine äußerst genaue und hochaufgelöste Bestimmung der Bewegungsdaten, nachdem der Zeitraum für Bewegungen bei der Aufnahme der Navigatordaten äußerst kurz gehalten wird. Dabei sei darauf hingewiesen, dass erfindungsgemäß auch ohne eine vollständige Aufnahme des Aufnahmebereichs, insbesondere des Kopfes, die Ermittlung hochaufgelöster Bewegungsdaten auch in Schichtrichtung/z-Richtung möglich ist, da sich mittels der SMS-Bildgebung dünne Fettnavigatorschichten anregen lassen und nicht wie bei aus dem Stand der Technik bekannten dreidimensionalen Fettnavigatoraufnahmen eine Slab-Anregung erfolgt, was bei wenig ausgelesenen Schichten zu Blurring-Effekten führen kann. Wird mithin eine bestimmte geforderte Auflösung vorausgesetzt, sind mittels der SMS-Bildgebung deutlich kürzere Fettnavigatorzeitabschnitte realisierbar. Beispielsweise können die Navigatordaten in Navigatorzeitabschnitten, die kürzer als 50 ms andauern, aufgenommen werden.

Zusammengefasst schlägt die vorliegende Erfindung mithin vor, mittels SMS-Bildgebung Navigatordaten simultan aus mehreren Fettnavigatorschichten aufzunehmen, um hochaufgelöste Daten auch bei geringer Schichtdicke aus dem Fettnavigator zu erhalten und Blurring wie bei dreidimensionalen Fettnavigatoren zu vermeiden, wobei eine äußerst große Anzahl simultan ausgelesener Fettnavigatorschichten möglich ist. Dadurch, dass die Fettnavigatorschichten zeitsimultan ausgelesen werden, befinden sich die ausgelesenen Fettnavigatorschichten im gleichen Bewegungszustand, was zu besonders hochqualitativen Navigatordaten und somit Bewegungsdaten führt.

Bezüglich der SMS-Bildgebung und deren konkreter Umsetzung wird bevorzugt das auch bei dem zitierten Artikel von Setsompop et al. beschriebene Verfahren eingesetzt, das bedeutet, die Fettnavigatorschichten werden gleichzeitig angeregt, während des Auslesens wird das Blipped CAIPIRINHA-Verfahren eingesetzt und die Navigatordaten werden durch Anwenden eines Schicht-GRAPPA-Algorithmus und eines in-plane-GRAPPA-Algorithmus ermittelt. Es sei angemerkt, dass die Kalibrierungsdaten für diese oder gegebenenfalls andere angewendete Algorithmen zur Trennung von Navigatordaten einzelner Fettnavigatorschichten bzw. zur Kompensation der in-plane-Unterabtastung im Rahmen der vorliegenden Erfindung auf unterschiedliche Weise gewonnen werden können. Beispielsweise ist es denkbar, Kalibrierungsdaten im ersten, dann gegebenenfalls etwas länger andauernden Navigatorzeitabschnitt vor dem ersten Aufnahmezeitabschnitt aufzunehmen und/oder Kalibrierungsdaten auch über mehrere Navigatorzeitabschnitte, bevorzugt mehrere erste Navigatorzeitabschnitte, zu sammeln. Insbesondere dann, wenn eine stärkere Bewegung anzeigende Bewegungsdaten vorliegen, ist es zudem möglich, eine Neuaufnahme von Kalibrierungsdaten und somit eine Neukalibrierung der Kerne der Algorithmen, insbesondere der GRAPPA-Algorithmen, anzustoßen. Es ist im Übrigen auch denkbar, Kalibrierungsdaten dieser Art auch als Referenzdaten für eine besonders zuverlässige Bestimmung der Bewegungsdaten zu nutzen, wie im Folgenden noch näher erläutert werden wird. Die Nutzung von Kalibrierungsdaten aus anderen Quellen ist ebenso denkbar, aber weniger bevorzugt.

In einer zweckmäßigen Weiterbildung der Erfindung kann vorgesehen sein, dass in dem Anregungsmodul eine Binomialpulsfolge zur fettselektiven Anregung verwendet wird. Die selektive Anregung der Fett-Spins kann mithin durch Multiband-Binomialpulse erfolgen, deren Pulsphasen so gestaltet sind, dass am Ende der Pulsfolge, also des Pulszuges, nur Magnetisierung von fettäquivalenten Spins in der Transversalebene vorhanden ist, während die Magnetisierung von wasseräquivalenten Spins in die Longitudinalebene zurückgeklappt wurde. Beispielsweise kann eine monopolare 121-Binomialpulsfolge verwendet werden, wobei der mittlere Hochfrequenzpuls mit umgekehrter Phase angewendet wird. Die Idee ist es hierbei, dass der erste Hochfrequenzpuls der Binomialpulsfolge die Magnetisierung von sowohl Fett-Spins als auch Wasser-Spins um denselben Flipwinkel auslenkt. Beide Spinarten haben zu diesem Zeitpunkt gleiche Phase. Aufgrund der leicht verschobenen Lamorfrequenzen für die Spinarten kann zu einem zweiten Zeitpunkt nach einer Wartezeit ein den doppelten Flipwinkel betreffender Hochfrequenzpuls der Binomialpulsfolge mit einer Phase von 180° angewandt werden. Hierdurch wird die Magnetisierung der Wasser-Spins wieder näher an die Longitudinalmagnetisierung geschoben, so dass nach einer weiteren Wartezeit, wenn die Spins wieder gleichphasig sind, durch einen letzten Hochfrequenzpuls der Binomialpulsfolge die Magnetisierung der Wasser-Spins wieder in die Longitudinalrichtung gedreht wird, während Magnetisierung der Fett-Spins zumindest Transversalanteile besitzt und somit zur Bildgebung genutzt werden kann.

Bei vielen gleichzeitig aufgenommenen Fettnavigatorschichten, was erfindungsgemäß ja auch bevorzugt ist, kann es aufgrund der Überlagerung vieler einzelner Hochfrequenzpulse (hoher Multibandfaktor) zu äußerst starken Hochfrequenzpulsen kommen, insbesondere zu einer Überschreitung der Leistungsfähigkeit der Sendekomponenten der Magnetresonanzeinrichtungen und/oder zur Überschreitung von SAR-Grenzwerten für den Patienten. Um dem entgegenzuwirken, kann ausgenutzt werden, dass Magnetresonanzsignale von fettäquivalenten Spins eine hohe Intensität aufweisen, so dass eine ausreichende Anregung auch bei kleineren Flipwinkeln gegeben ist. Mithin kann der Flipwinkel der Binomialpulsfolge reduziert werden, so dass beispielsweise vorgesehen sein kann, dass der Flipwinkel der gesamten Binomialpulsfolge kleiner als 30°, insbesondere kleiner als 20°, gewählt wird. So werden zu hohe Leistungen bei einzelnen Hochfrequenzpulsen vermieden.

Alternativ oder zusätzlich zu einer solchen Reduzierung des Flipwinkels können auch andere Vorgehensweisen eingesetzt werden, um die Leistung für Einzelpulse im Anregungsmodul gering zu halten. In diesem Kontext sieht eine zweckmäßige erweiterung der vorliegenden Erfindung vor, dass Binomialpulsfolgen für unterschiedliche Fettnavigatorschichten derart versetzt ausgegeben werden, dass wenigstens ein Hochfrequenzpuls einer Binomialpulsfolge zwischen zwei Hochfrequenzpulsen der anderen Binomialpulsfolge ausgegeben wird. Das bedeutet, die Zeit zwischen zwei Hochfrequenzpulsen der einen Binomialpulsfolge wird verwendet, um Pulse einer zweiten Binomialpulsfolge auszugeben, so dass unterschiedliche Fettnavigatorschichten mit Binomialpulsfolgen angeregt werden, wie dies beispielsweise in der nachveröffentlichten deutschen Patentanmeldung DE 10 2018 201 810.3 der Anmelderin vorgeschlagen wurde. Konkret wird dort ein Verfahren zum simultanen Aufnehmen von Magnetresonanzsignalen aus einem Untersuchungsbereich mit zwei unterschiedlichen Gewebearten in mehreren Schichten vorgeschlagen, aufweisend die folgenden Schritte:
- Einstrahlen einer ersten Binomialpulsfolge zum Anregen von zumindest einer ersten Schicht, wobei die Flipwinkel der Hochfrequenzpulse in der ersten Binomialpulsfolge so gewählt sind, dass nach Ende der Einstrahlung der ersten Binomialpulsfolge eine der beiden Gewebearten im Wesentlichen keine Transversalmagnetisierung aufweist, während die andere der beiden Gewebearten eine Transversalmagnetisierung aufweist, und wobei ein Hochfrequenzpuls der ersten Binomialpulsfolge zu einem Zeitpunkt eingestrahlt wird, bei dem die zwei unterschiedlichen Gewebearten eine entgegengesetzte Phasenlage haben, wobei alle Hochfrequenzpulse der ersten Binomialpulsfolge während einer Schaltung von Magnetfeldgradienten eingestrahlt werden, die jeweils eine erste Polarität aufweisen,

- Einstrahlen einer zweiten Binomialpulsfolge zum Anregen von zumindest einer zweiten Schicht, wobei die Flipwinkel der Hochfrequenzpulse in der zweiten Binomialpulsfolge so gewählt sind, dass nach Ende der Einstrahlung der zweiten Binomialpulsfolge die eine Gewebeart im Wesentlichen keine Transversalmagnetisierung aufweist, während die andere Gewebeart eine Transversalmagnetisierung aufweist, und wobei ein Hochfrequenzpuls der zweiten Binomialpulsfolge zu einem Zeitpunkt eingestrahlt wird, bei dem die zwei unterschiedlichen Gewebearten eine entgegengesetzte Phasenlage haben, wobei alle Hochfrequenzpulse der zweiten Binomialpulsfolge während einer Schaltung von den Magnetfeldgradienten eingestrahlt werden, die alle eine zweite Polarität aufweisen, wobei zumindest ein Hochfrequenzpuls der zweiten Binomialpulsfolge zeitlich zwischen zwei Hochfrequenzpulsen der ersten Binomialpulsfolge eingestrahlt wird,
- gleichzeitiges Auslesen der Magnetresonanzsignale aus der zumindest einen ersten und der zumindest einen zweiten Schicht.

Ein derartiges Vorgehen erweist sich auch bezüglich des Fettnavigators der vorliegenden Erfindung als zweckmäßig.

Allgemein sei an dieser Stelle nochmals darauf hingewiesen, dass die Fettnavigatorschichten eines Navigatorzeitabschnitts den Aufnahmebereich nicht komplett abdecken müssen, sondern bevorzugt dünn gewählt werden und den Aufnahmebereich in Schichtrichtung mithin "unterabtasten". Nichtsdestotrotz werden in Schichtrichtung aufgrund der dünnen Schichten hochgenaue Informationen geliefert, wobei wiederum die räumliche Spärlichkeit von Fett ausgenutzt wird.

In konkreten Ausführungsbeispielen der vorliegenden Erfindung kann vorgesehen sein, dass die Bewegungsdaten durch Vergleich der aktuell aufgenommen Navigatordaten mit einen vorherigen Bewegungszustand zeigenden Referenzdaten ermittelt werden, wobei die Navigatordaten zu den Referenzdaten registriert werden. Dabei ist es besonders vorteilhaft, wenn die Referenzdaten Navigatordaten wenigstens eines vorangehenden Navigatorzeitabschnitts umfassen.

Mithin sieht eine erste, einfache Ausgestaltung der vorliegenden Erfindung vor, dass in jedem Navigatorzeitabschnitt dieselben Fettnavigatorschichten vermessen werden und auf die in dem vorherigen Navigatorzeitabschnitt aufgezeichneten Fettnavigatorschichten registriert werden. Um hierbei möglichst genaue Bewegungsdaten zu erhalten, ist es besonders vorteilhaft, wenn eine große Zahl an Fettnavigatorschichten simultan aufgenommen wird, insbesondere mehr als acht Fettnavigatorschichten, die den Aufnahmebereich möglichst gleichmäßig abdecken.

Eine bevorzugte Ausführungsform der vorliegenden Erfindung kann vorsehen, dass für wenigstens einen Navigatorzeitabschnitt, insbesondere wenigstens einen zeitlich ersten Navigatorzeitabschnitt, eine größere Zahl von Fettnavigatorschichten zur Erzeugung der Referenzdaten aufgenommen werden. In einer derartigen zweiten Ausgestaltung der vorliegenden Erfindung kann vorgesehen sein, dass initial in einem ersten Navigatorzeitabschnitt ein Referenzdatensatz aufgenommen wird, der eine höhere Zahl an Fettnavigatorschichten umfasst, insbesondere in mehreren Repetitionen, beispielsweise durch simultane Aufnahme von vier ersten Fettnavigatorschichten in einer ersten Repetition und durch Aufnahme von vier zweiten Fettnavigatorschichten in einer zweiten Repetition. In weiteren Navigatorzeitabschnitten werden dann weniger, beispielsweise nur noch vier, Fettnavigatorschichten aufgenommen und jeweils auf diesen Referenzdatensatz räumlich registriert, um die Bewegungsdaten zu ermitteln. Auf diese Weise liegt eine genauere Referenz vor. Es sei darauf hingewiesen, dass es grundsätzlich auch denkbar ist, die Referenzdaten im ersten Navigatorzeitabschnitt ohne SMS-Bildgebung auch zur Nutzung als Kalibrierungsdaten, beispielsweise zur Kalibrierung von Kernen angewendeter GRAPPA-Algorithmen, zu ermitteln.

In einer anderen vorteilhaften Weiterbildung der vorliegenden Erfindung kann auch vorgesehen sein, dass die Referenzdaten aus mehreren zeitlich aufeinanderfolgenden Navigatorzeitabschnitten, in denen unterschiedliche Fettnavigatorschichten simultan aufgenommen werden, zusammengestellt werden. Das bedeutet, aus der Zusammenschau mehrerer in aufeinanderfolgenden Navigatorzeitabschnitten aufgenommenen Schichtgruppen von Fettnavigatorschichten kann ein Referenzdatensatz synthetisiert werden, auf den nachfolgende Navigatordaten registriert werden, um die Bewegungsdaten zu bestimmen. Hierbei wird vorteilhaft ein länger andauernder initialer Scan von Referenzdaten eingespart und Relaxationseffekte werden abgeschwächt, insbesondere dann, wenn aufzunehmende Fettnavigatorschichten passend mit den Bildgebungschichten der Aufnahmeabschnitte verschachtelt werden. In einer solchen Ausgestaltung werden die Referenzdaten bevorzugt immer aktuell gehalten, so dass sich die Abweichung immer auf den vorangehenden Navigatorzeitabschnitt bezieht. Bei einer Aufnahme von Referenzdaten in einem ersten Navigatorzeitabschnitt kann es dahingegen zweckmäßig sein, nach zu starker Bewegung, beschrieben durch die Bewegungsdaten, eine erneute Aufnahme von Referenzdaten zu triggern.

Im Rahmen der vorliegenden Erfindung werden wenigstens zeitweise in unterschiedlichen Navigatorzeitabschnitten, insbesondere jeweils abwechselnd, eine unterschiedlicher Schichtgruppe von Fettnavigatorschichten aufgenommen.

Zweckmäßigerweise können bei zwei abwechselnd aufgenommenen Fettnavigatorschichtgruppen diese ineinander verschachtelt sein. Die Aufzeichnung anderer Schichtgruppen von Fettnavigatorschichten bei aufeinanderfolgenden Navigatorzeitabschnitten hat den Vorteil, dass Relaxationseffekte abgeschwächt werden, da nicht immer die gleichen Fettnavigatorschichten angeregt werden.

In einer dritten Ausgestaltung des erfindungsgemäßen Verfahrens kann dann, wenn in einem ersten Navigatorzeitabschnitt ein Referenzdatensatz aufgenommen wurde, beispielsweise vorgesehen sein, dass immer abwechselnd eine andere Schichtgruppe von Fettnavigatorschichten in den folgenden Navigatorzeitabschnitten aufgezeichnet werden und auf den Referenzdatensatz registriert werden, insbesondere jeweils gegeneinander versetzte, also ineinander verschachtelte Fettnavigatorschichten aufweisende Schichtgruppen.

In einer besonders vorteilhaften vierten Ausgestaltung der vorliegenden Erfindung kann auf das Erstellen des initialen Referenzdatensatzes verzichtet werden, nachdem in unterschiedlichen Repetitionen, insbesondere also Navigatorzeitabschnitten, unterschiedliche Schichtgruppen von Fettnavigatorschichten aus dem Aufnahmebereich ausgelesen werden. Auch hier kann beispielsweise eine abwechselnde Aufnahme von ineinander verschachtelten Schichtgruppen erfolgen. Dann kann, im Beispiel aus zwei aufeinanderfolgenden Navigatorzeitabschnitten, ein gerade aktueller Referenzdatensatz bezogen auf den Aufnahmebereich abgeleitet werden, woraufhin die nachfolgenden Navigatordaten auf das gerade aktuelle Referenzvolumen registriert werden. Auf diese Weise kann neben der Abschwächung von Relaxationseffekten erreicht werden, dass eine initiale, länger andauernde Aufnahme von Referenzdaten eingespart wird.

In einer besonders vorteilhaften Weiterbildung der vorliegenden Erfindung kann zudem vorgesehen sein, dass die Schichtorientierung der Fettnavigatorschichten wenigstens einer der Schichtgruppen gegen die Schichtorientierung der Fettnavigatorshichten wenigstens einen anderen der Schichtgruppen gedreht ist, insbesondere um 90°. So kann beispielsweise in einer fünften konkreten Ausgestaltung der vorliegenden Erfindung vorgesehen werden, dass in jedem Navigatorzeitabschnitt eine andere Schichtorientierung der Fettnavigatorschichten gewählt wird, was eine bessere räumliche Registrierung bei einer (gegebenenfalls) geringeren Anzahl an gleichzeitig ausgelesenen Fettnavigatorschichten erlaubt. Der dieser Ausgestaltung zugrunde liegende Gedanke ist, dass die Fettnavigatorschichten in vielen Fällen den Aufnahmebereich nicht vollständig abdecken, so dass beispielsweise Schwierigkeiten bei der Registrierung bei Bewegungen in Schichtrichtung auftreten können, wenn zu den nun aufgenommenen Navigatordaten beispielsweise noch keine passenden Referenzdaten vorliegen. Derartige Bewegungen können jedoch durch Schichten in anderen Richtungen hervorragend und deutlich besser erfasst werden, was die Qualität der Bewegungsdaten und mithin deren Genauigkeit deutlich erhöht. Dabei werden bevorzugt jeweils die verschiedenen Orientierungen aufeinanderfolgender Navigatorzeitabschnitte in die Registrierung einbezogen.

Die Bewegungsdaten können hinsichtlich der Magnetresonanzdaten für eine retrospektive oder eine prospektive Bewegungskorrektur genutzt werden. Bei einer retrospektiven Bewegungskorrektur erfolgt letztlich eine Korrektur erst bei der Ermittlung von Magnetresonanzbilddatensätzen aus den Magnetresonanzdaten, denen jeweils ein Bewegungszustand zugeordnet ist. Beispielsweise können Teilbilder für die verschiedenen Bewegungszustände des Aufnahmebereichs ermittelt und dann unter Berücksichtigung der Bewegungsdaten zusammengeführt werden. Bei einer prospektiven Korrektur können unmittelbar Parameter der zur Aufnahme der Magnetresonanzdaten verwendeten Magnetresonanzsequenz angepasst werden, um Bewegungseffekte schon bezüglich der aufgenommenen Magnetresonanzsignale zu kompensieren und somit eine weniger aufwändige Auswertung zu erreichen.

Die Magnetresonanzdaten werden in zwischen Navigatorzeitabschnitten liegenden Aufnahmezeitabschnitten aufgenommen. Dabei sind Ausgestaltungen denkbar, in denen lediglich eine Ausspielung der Magnetresonanzsequenz, mithin eine zeitliche Repetition, zwischen zwei Navigatorzeitabschnitten liegt; üblicherweise werden jedoch mehrere Repetitionen zwischen zwei Navigatorzeitabschnitten liegen können. So kann vorgesehen sein, dass in den Aufnahmezeitabschnitten immer wenigstens zwei Anwendungen einer Magnetresonanzsequenz erfolgen, mithin zwei oder mehr Repetitionen dort liegen. Dabei können zur Aufnahme der Magnetresonanzdaten selbstverständlich auch unterschiedliche Magnetresonanzsequenzen eingesetzt werden, insbesondere, wenn unterschiedliche Arten von Magnetresonanzbilddatensätzen des Aufnahmebereichs nach Rekonstruktion erhalten werden sollen.

Vorzugsweise können die Magnetresonanzdaten mit einer Fettsignale unterdrückenden Magnetresonanzsequenz, insbesondere einer EPI-Sequenz (EPI - Echo Planar Imaging), aufgenommen werden. Während sich die hier vorgestellte Navigatormethode grundsätzlich mit allen bildgebenden Magnetresonanzsequenzarten kombinieren lässt, ist der Einsatz der hier vorgestellten SMS-Fettnavigatoren besonders nützlich bei Magnetresonanzsequenzen, welche eine Fettsättigungsmodul beinhalten, beispielsweise EPI-Sequenzen oder fettgesättigten TSE-Sequenzen, weil die beeinflussten Fett-Spins für die Bildgebung ohnehin unterdrückt werden sollen und es somit zu vernachlässigbarem Cross-Talk bzw. vernachlässigbaren Magnetisierungseffekten kommen sollte. Bei Magnetresonanzsequenzen zur Aufnahme der Magnetresonanzdaten, die ohne eine Unterdrückung der Magnetresonanzsignale von Fett-Spins arbeiten, kann, wie oben beschrieben, der Flipwinkel im Anregungsmodul, insbesondere der Flipwinkel der Binomialpulsfolge, reduziert werden, um nicht nur die Spitzenleistung zu reduzieren, sondern auch den Einfluss des Fettnavigators auf die Magnetresonanzsequenz zu minimieren.

Vorzugsweise kann die Fettnavigatorsequenz eine EPI-Sequenz ohne Refokussierungspulse sein. Das bedeutet, insbesondere wird nach der Anregung der Fett-Spins im Anregungsmodul ein bevorzugt durch eine in-plane-Beschleunigungstechnik beschleunigter EPI-Auslesezug verwendet. Hierdurch wird das Ausspielen von Refokussierungspulsen mit (aufgrund der vielen Fettnavigatorschichten) hoher Spitzenleistung vermieden. Es sind jedoch auch andere Ausgestaltungen denkbar, in denen beispielsweise eine Refokussierung mittels PINS-Pulsen erfolgt.

Neben dem Verfahren betrifft die vorliegende Erfindung auch eine Magnetresonanzeinrichtung, aufweisend eine zur Durchführung des erfindungsgemäßen Verfahrens ausgebildete Steuereinrichtung. Sämtliche Ausführungen bezüglich des erfindungsgemäßen Verfahrens lassen sich analog auf die erfindungsgemäße Magnetresonanzeinrichtung übertragen, so dass auch mit dieser die bereits genannten Vorteile erhalten werden können.

Die Steuereinrichtung, welche üblicherweise wenigstens einen Prozessor und/oder wenigstens ein Speichermittel umfasst, kann hierbei neben einer Sequenzeinheit, die die Ausgabe von Sequenzen und die Aufnahme von Magnetresonanzsignalen steuert, wenigstens eine Auswertungseinheit aufweisen, mittels derer die Bewegungsdaten ermittelt werden können. Ferner kann eine Korrektureinheit vorgesehen sein, über die eine prospektive und/oder retrospektive Korrektur der Magnetresonanzdaten hinsichtlich auftretender Bewegungen im Aufnahmebereich, insbesondere dem Kopf eines Patienten, erfolgen kann.

Ein erfindungsgemäßes Computerprogramm ist beispielsweise direkt in einen Speicher einer Steuereinrichtung einer Magnetresonanzeinrichtung ladbar und weist Programmmittel auf, um die Schritte eines erfindungsgemäßen Verfahrens auszuführen, wenn das Computerprogramm in der Steuereinrichtung der Magnetresonanzeinrichtung ausgeführt wird. Das Computerprogramm kann auf einem erfindungsgemäßen elektronisch lesbaren Datenträger gespeichert sein, welcher mithin darauf gespeicherte elektronisch lesbare Steuerinformationen umfasst, welche zumindest ein erfindungsgemäßes Computerprogramm umfassen und derart ausgestaltet sind, dass sie bei Verwendung des Datenträgers in einer Steuereinrichtung einer Magnetresonanzeinrichtung ein erfindungsgemäßes Verfahren durchführen. Bei dem Datenträger kann es sich insbesondere um einen nichttransienten Datenträger, beispielsweise eine CD-ROM, handeln. Weitere Vorteile und Einzelheiten der vorliegenden Erfindung ergeben sich aus den im Folgenden beschriebenen Ausführungsbeispielen sowie anhand der Zeichnung. Dabei zeigen:
- Fig. 1: einen Ablaufplan zur Aufnahme von Navigatordaten mit einer Fettnavigatorsequenz,
- Fig. 2: eine verwendbare Binomialpulsfolge,
- Fig. 3: die Auswirkungen der Binomialpulsfolge der Fig. 2 auf Magnetisierungen von Wasser- und Fett-Spins,
- Fig. 4: einen zeitlichen Versatz von Binomialpulsfolgen,
- Fig. 5: ein Schema zur Erläuterung eines Beispiels welches nicht Teil der beanspruchten Erfindung ist,

- Fig. 6: ein Schema zur Erläuterung eines ersten Ausführungsbeispiels des erfindungsgemäßen Verfahrens,
- Fig. 7: ein Schema zur Erläuterung eines zweiten Ausführungsbeispiels des erfindungsgemäßen Verfahrens,
- Fig. 8: ein Schema zur Erläuterung eines dritten Ausführungsbeispiels des erfindungsgemäßen Verfahrens,
- Fig. 9: ein Schema zur Erläuterung eines vierten Ausführungsbeispiels des erfindungsgemäßen Verfahrens, und
- Fig. 10: eine Prinzipskizze einer erfindungsgemäßen Magnetresonanzeinrichtung.

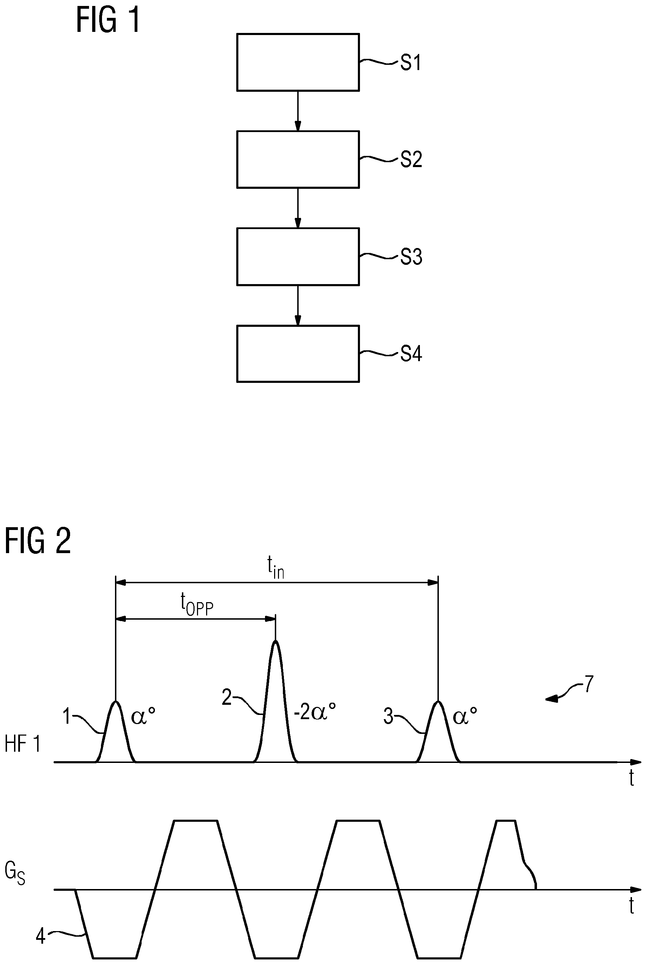

Fig. 1 erläutert einen prinzipiellen Ablaufplan zur Aufnahme und Ermittlung von Navigatordaten mittels einer Fettnavigatorsequenz im erfindungsgemäßen Verfahren. Dabei sollen durch Verwendung von SMS-Bildgebung simultan Magnetresonanzsignale aus mehreren Fettnavigatorschichten aufgenommen werden, um hieraus die Navigatordaten zu ermitteln. Hierzu wird in einem Anregungsmodul in einem Schritt S1 eine Hochfrequenzpulsfolge ausgegeben, die auf die mehreren Fettnavigatorschichten wirkt und selektiv Fett-Spins anregt. Dafür werden vorliegend in dem Anregungsmodul im Schritt S1 Binomialpulsfolgen verwendet.

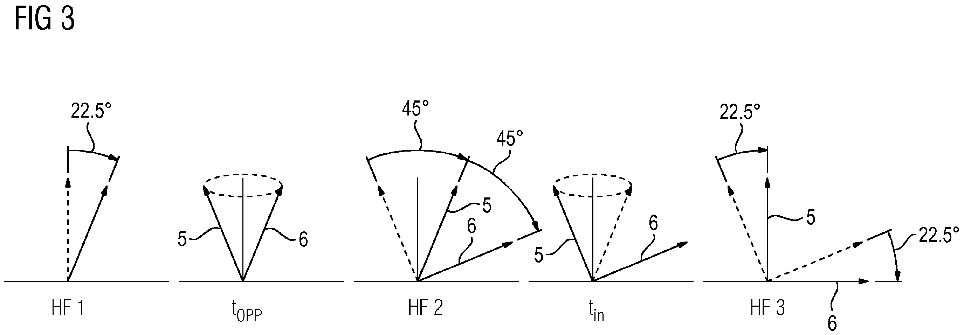

Eine fettselektive Binomialpulsfolge sei im Hinblick auf Fig. 2 und Fig. 3 näher erläutert. Die dort dargestellte Binomialpulsfolge 7 ist eine 1-2-1 Binomialpulsfolge, wobei sich die angegebenen Binomialkoeffizienten auf den Flipwinkel, in der Figur a, beziehen. Dabei wird zunächst ein Hochfrequenzpuls 1 in den Aufnahmebereich, konkret die Fettnavigatorschichten, eingestrahlt, wobei der Aufnahmebereich zwei unterschiedliche Gewebekomponenten bzw. Spinarten aufweist, vorliegend Fett-Spins und Wasser-Spins. Der erste Hochfrequenzpuls 1 wirkt sowohl auf die Fett-Spins wie auch auf die Wasser-Spins und lenkt die Magnetisierung, wie in Fig. 2 dargestellt ist, um den Flipwinkel a, im Beispiel 22,5°, aus der Longitudinalrichtung aus, wobei die Fett-Spins und die Wasser-Spins zunächst noch in Phase sind. Nach einer Wartezeit t_{OPP} haben die Fett- und Wasser-Spins jedoch eine entgegengesetzte Phasenlage, wie es in Fig. 2 durch die Magnetisierung 5 bzw. 6 angedeutet ist.

Wird nun der Hochfrequenzpuls 2 mit einem doppelten Flipwinkel und umgedrehter Phasenlage ausgegeben, werden die Magnetisierungen 5 und 6, wie in Fig. 3 gezeigt, weitergekippt. Nach einer weiteren Wartezeit t_{OPP}, das heißt insgesamt nach der Zeitspanne tᵢₙ, wird der dritte Hochfrequenzpuls 3 wiederum mit dem Flipwinkel α eingestrahlt, so dass insgesamt der Magnetisierungsvektor 5 für Wasser für keine Transversalmagnetisierung aufweist, während der Magnetisierungsvektor 6 für Fett in der Transversalebene liegt. Eine derartige Binomialpulsfolge 7 bzw. die sich nach dieser Binomialpulsfolge 7 ergebenden Magnetisierungen 5, 6 können dann im Schritt S2 mit einer üblichen Signalauslese, insbesondere durch einen Auslesezug einer EPI-Sequenz, kombiniert werden. Da nur die Magnetisierung 6 in der Transversalebene liegt, hat die andere Magnetisierung 5, hier das Wassersignal, keinen Signalanteil. Es sei darauf hingewiesen, dass der Erläuterung halber in Fig. 3 auch der Verlauf 4 des Schichtselektionsgradienten G_{S} gezeigt ist.

Für mehrere Fettnavigatorschichten, wobei aufgrund der räumlichen Spärlichkeit von Fett, insbesondere bei den Kopf des Patienten betreffenden Aufnahmebereichen, eine große Anzahl gewählt werden kann, müssten grundsätzlich entsprechend dem Multibandfaktor (Zahl der Fettnavigatorschichten für eine Fettnavigatorsequenz) Binomialpulsfolgen 7 überlagert werden, wodurch es zu hohen Spitzenleistungen kommen kann, insbesondere bezüglich des zentralen Hochfrequenzpulses 2. Um dem entgegenzuwirken, können erfindungsgemäß zwei Maßnahmen vorgesehen werden, nämlich zum eine Reduzierung des Flipwinkels a, so dass sich beispielsweise für die Magnetisierung 6 statt einem Gesamtflipwinkel von 90° nur ein kleinerer Gesamtflipwinkel, beispielsweise von 20°, ergibt. Nachdem Fettsignale eine hohe Intensität aufweisen, ist eine ausreichende Anregung auch bei kleineren Flipwinkeln gegeben.

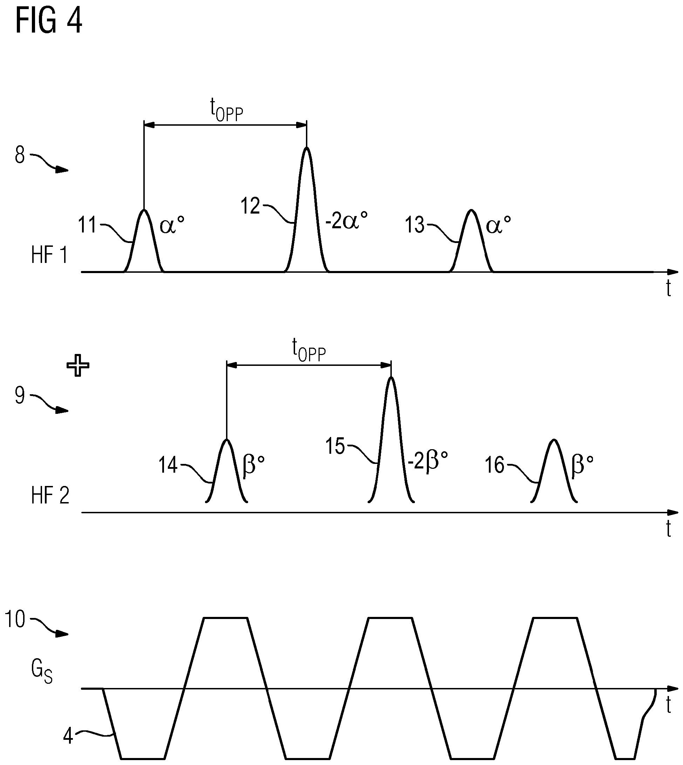

Zum anderen kann jedoch eine zeitliche Verschiebung von Hochfrequenzpulsen, die unterschiedliche Fettnavigatorschichten anregen, eingesetzt werden, wie dies in Bezug auf Fig. 4 näher erläutert wird.

Gezeigt sind dort eine erste Binomialpulsfolge 8 und eine zweite Binomialpulsfolge 9 sowie wiederum der Verlauf 10 des Schichtselektionsgradienten G_{S}. Die erste Binomialpulsfolge 8 und die zweite Binomialpulsfolge 9 sind wiederum 1-2-1-Binomialpulsfolgen mit Hochfrequenzpulsen 11, 12, 13 bzw. 14, 15, 16, wobei die erste Binomialpulsfolge einen Flipwinkel a, die zweite Binomialpulsfolge einen Flipwinkel β benutzt. Die zeitlichen Abstände sind immer so gewählt, dass die Magnetisierung der Wasser-Spins nach ihrer Anwendung in den entsprechenden Fettnavigatorschichten Null beträgt. Ersichtlich werden die Hochfrequenzpulse 11 bis 13 bzw. 14 bis 16 zeitlich versetzt geschaltet, so dass der Hochfrequenzpuls 14 zeitlich zwischen den Hochfrequenzpulsen 11 und 12 zum Liegen kommt und der Hochfrequenzpuls 15 zwischen den Hochfrequenzpulsen 12 und 13. So können Spitzenleistungen weiter reduziert werden.

In einem Schritt S2, vgl. wiederum Fig. 1, folgt dann ein Auslesemodul, wobei vorliegend ein durch eine in-plane-Beschleunigungstechnik beschleunigter EPI-Auslesezug verwendet wird, um das Ausspielen von Refokussierungspulsen mit hoher Spitzenleistung zu vermeiden. In einem Schritt S3 werden dann die aufgenommenen Navigatordaten durch Anwendung eines Schicht-GRAPPA-Algorithmus den verschiedenen Fettnavigatorschichten zugeordnet, während in einem Schritt S4 ein in-plane-GRAPPA-Algorithmus bezüglich der Unterabtastung in der Schichtebene eingesetzt wird. Nach dem Schritt S4 liegen somit Navigatordaten in den jeweiligen Fettnavigatorschichten vor.

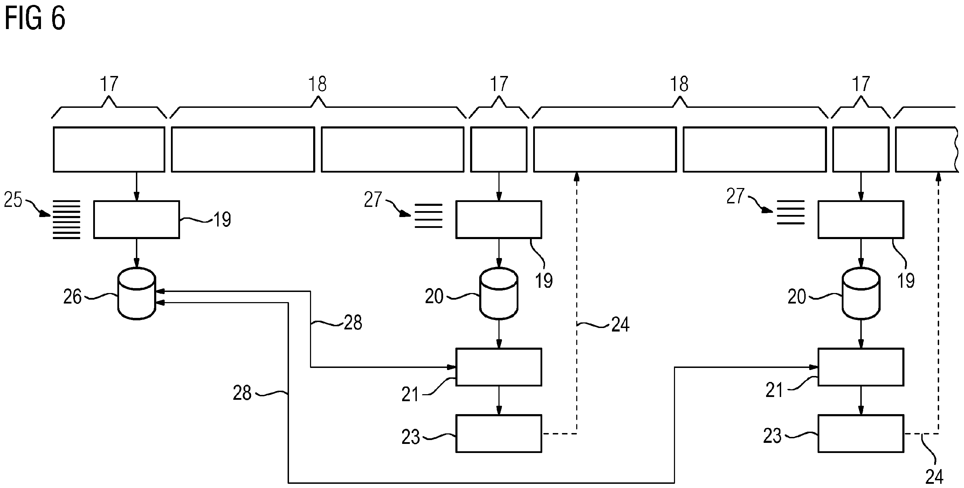

Die Figuren 6 bis 9 zeigen nun konkrete Ausgestaltungen des erfindungsgemäßen Verfahrens bei der Aufnahme von Magnetresonanzdaten mit wenigstens einer Magnetresonanzsequenz. Dabei ist in all diesen Figuren im oberen Bereich die zeitliche Abfolge von Navigatorzeitabschnitten 17 und Aufnahmezeitabschnitten 18 gezeigt. Die Verwendung von zwei Repetitionen in den Aufnahmezeitabschnitten ist rein beispielhaft zu verstehen; insbesondere können auch mehr Repetitionen innerhalb der Aufnahmezeitabschnitte liegen.

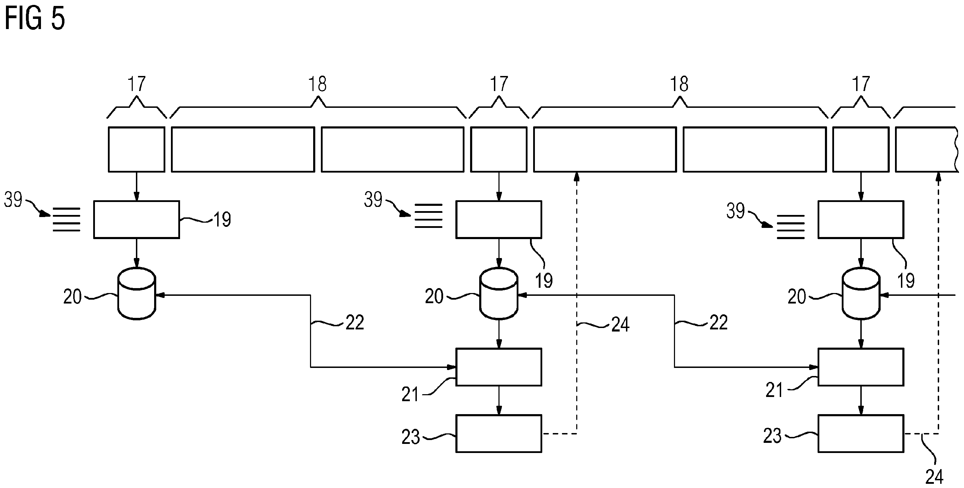

Im in Fig. 5 dargestellten Beispiel, welches nicht Teil der beanspruchten Erfindung ist, werden in jedem gezeigten Navigatorzeitabschnitt 17 in der gleichen Schichtgruppe 39 von Fettnavigatorschichten simultan Magnetresonanzsignale aufgenommen und in jeweiligen Schritten 19 die Navigatordaten 20 rekonstruiert, wie bezüglich Fig. 1 beschrieben. Dabei dienen die im jeweils vorangehenden Navigatorzeitabschnitt 17 aufgenommenen Navigatordaten 20 vorliegend auch als Referenzdaten, nachdem zur Ermittlung der Bewegungsdaten im Schritt 21 jeweils eine Registrierung mit den zuvor aufgenommenen Navigatordaten 20 erfolgt, vgl. Pfeil 22. Die so ermittelten Bewegungsdaten werden gemäß dem Schritt 23 zur Korrektur genutzt, und zwar entweder zur retrospektiven Korrektur oder aber bevorzugt, wie durch den gestrichelten Pfeil 24 angedeutet, zur prospektiven Bewegungskorrektur, die unmittelbar Auswirkungen auf die Bildgebung im nächsten Aufnahmezeitabschnitt 18 hat.

Das hier dargestellte, einfache Beispiel ist insbesondere dann hervorragend anwendbar, wenn eine große Zahl von Fettnavigatorschichten in der Schichtgruppe 39 enthalten ist.

Fig. 6 erläutert ein erstes Ausführungsbeispiel des erfindungsgemäßen Verfahrens, wobei im Unterschied zum vorigen Beispiel, welches nicht Teil der beanspruchten Erfindung ist, im ersten, ganz links mit verlängerter Zeitdauer angezeigten Navigatorzeitabschnitt 17 in mehreren Repetitionen Magnetresonanzsignale aus einer größeren Anzahl von Fettnavigatorschichten einer ersten Schichtgruppe 25 aufgenommen werden, aus denen im Schritt 19 Referenzdaten 26 ermittelt werden. Beispielsweise ist es denkbar, im beispielhaft dargestellten Beispiel von acht Navigatorschichten der ersten Schichtgruppe 25 zwei SMS-Bildgebungsrepetitionen für zwei gegeneinander versetzte Schichtgruppen von Fettnavigatorschichten durchzuführen, die gemeinsam die erste Schichtgruppe 25 ergeben. Möglich ist es aber auch, wenn die Referenzdaten 26 auch als Kalibrierungsdaten, insbesondere für Kerne der verwendeten GRAPPA-Algorithmen, dienen sollen, diese ohne SMS-Bildgebung aufzunehmen, das resultierende Verfahren wäre dann jedoch nicht Teil der beanspruchten Erfindung.

Die Referenzdaten 26, die den Aufnahmebereich vollständig abdecken, enthalten mithin auch Informationen zu zwischen Fettnavigatorschichten der später aufzunehmenden zweiten Schichtgruppen 27 liegenden Fettanteilen. Dies erhöht die Verlässlichkeit der wiederum in einem Schritt 21 vorgenommenen Registrierung der in späteren Navigationszeitabschnitten 17 erhaltenen Navigatordaten 20 jeweils mit dem Referenzdatensatz 26, vgl. Pfeile 28. Die zusätzliche Information in den Referenzdaten 26 ist dabei insbesondere bei Bewegungen in Schichtrichtung nützlich.

Sollten zu einem Zeitpunkt während der Gesamtaufnahmezeit zu starke Bewegungen aufgetreten sein, kann im Übrigen in einem weiteren verlängerten Navigatorzeitabschnitt 17 eine Neuaufnahme der Referenzdaten 26 erfolgen.

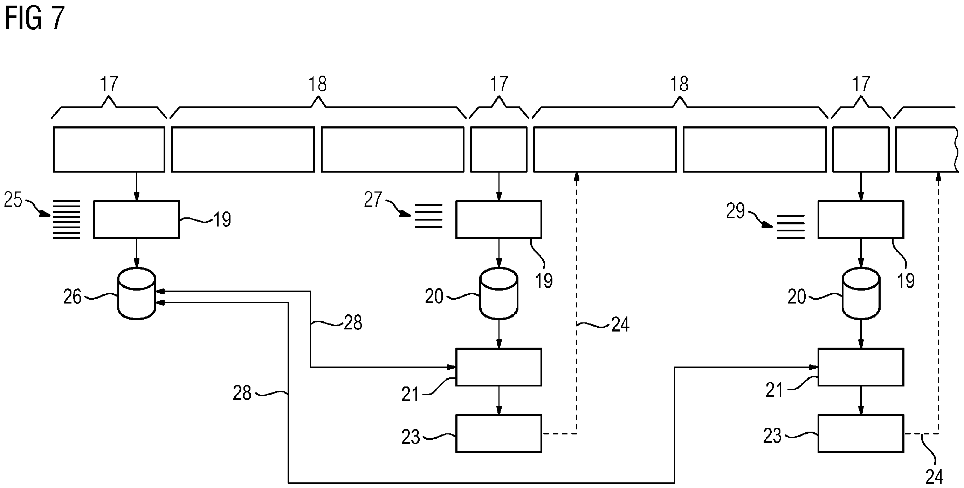

Das durch Fig. 7 erläuterte zweite Ausführungsbeispiel unterscheidet sich von dem ersten Ausführungsbeispiel dahingehend, dass nicht in jedem der weiteren Navigatorzeitabschnitte 17 dieselben vier Schichten der Schichtgruppe 27 aufgenommen werden, sondern alternierend jeweils vier der acht Schichten der Schichtgruppe 25 aufgenommen werden, das bedeutet, es werden abwechselnd die Fettnavigatorschichten der Schichtgruppe 27 und die Fettnavigatorschichten der aus den übrigen Fettnavigatorschichten der Schichtgruppe 25 gebildeten Schichtgruppe 29 aufgenommen. Das hat den Vorteil, dass Relaxationseffekte abgeschwächt sind, da immer unterschiedliche Fettnavigatorschichten angeregt werden.

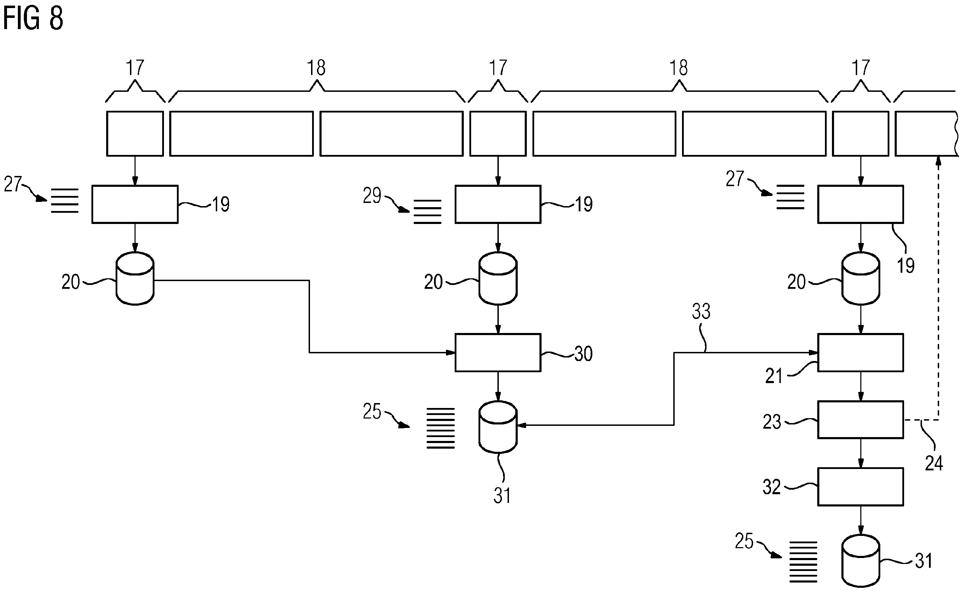

Im dritten Ausführungsbeispiel gemäß Fig. 8 erfolgt ebenso alternierend eine Vermessung der Schichtgruppen 27 und 29, die im Übrigen als ineinander verschachtelt verstanden werden können, wobei dort allerdings vorgesehen ist, nicht zu Beginn Referenzdaten 26 aufzunehmen, sondern im zweiten Navigationszeitabschnitt 17, wenn Navigatordaten 20 aus beiden Schichtgruppen 27 und 29 vorliegen, hieraus in einem Schritt 30 Referenzdaten für die die Schichtgruppen 27 und 29 zusammenfassende Schichtgruppe 25 zu ermitteln. Die Referenzdaten 31 werden mit jedem Navigationszeitabschnitt 17 gemäß dem Schritt 32 aktualisiert. Zudem dienen die jeweils aktuellen Referenzdaten 31 zur Registrierung, vgl. Pfeil 33, mit den aktuellen Navigatordaten 20, zum hieraus entsprechend die Bewegungsdaten abzuleiten und eine entsprechende Korrektur vornehmen zu können.

Dabei sei angemerkt, dass die alternierende Aufnahme zweier Schichtgruppen 27, 29 nicht beschränkend ist, sondern durchaus auch mehr als die zwei Schichtgruppen 27, 29 zyklisch durchlaufen werden können, beispielsweise drei oder vier unterschiedliche Schichtgruppen.

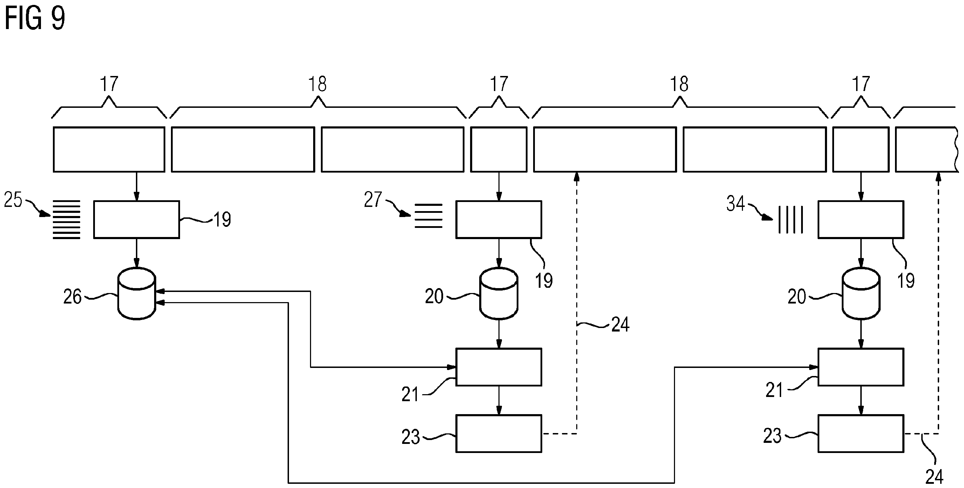

Fig. 9 zeigt schließlich ein viertes Ausführungsbeispiel des erfindungsgemäßen Verfahrens, welches sich von dem zweiten Ausführungsbeispiel darin unterscheidet, dass alternierend Schichtgruppen 27, 34 mit unterschiedlicher Orientierung der Fettnavigatorschichten aufgenommen werden. Dies hat den Vorteil einer verbesserten räumlichen Registrierung, gerade bei einer geringeren Anzahl gleichzeitig ausgelesener Fettnavigatorschichten.

Es sei darauf hingewiesen, dass selbstverständlich auch die Ausführung gemäß Fig. 8, betreffend die regelmäßige Aktualisierung der Referenzdaten 31, mit der vierten Ausgestaltung der Fig. 9 kombiniert werden kann.

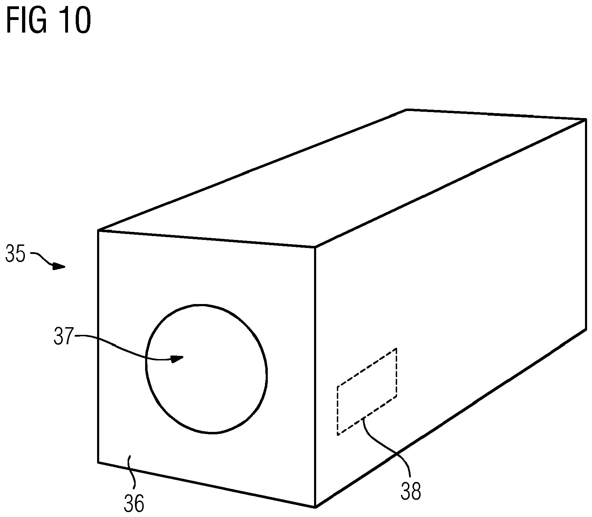

Fig. 10 zeigt schließlich eine Prinzipskizze einer erfindungsgemäßen Magnetresonanzeinrichtung 35. Diese weist, wie grundsätzlich bekannt, eine Hauptmagneteinheit 36 mit einer darin definierten Patientenaufnahme 37 auf, in die mittels einer hier nicht näher gezeigten Patientenliege ein Patient eingefahren werden kann. Die Patientenaufnahme 37 umgebend können eine Hochfrequenzspulenanordnung und eine Gradientenspulenanordnung der Magnetresonanzeinrichtung 35 vorgesehen sein.

Der Betrieb der Magnetresonanzeinrichtung 35 wird durch eine Steuereinrichtung 38 gesteuert, die auch zur Durchführung des erfindungsgemäßen Verfahrens ausgebildet ist. Hierzu kann die Steuereinrichtung 38 beispielsweise eine entsprechend angepasste Sequenzeinheit, eine entsprechend angepasste Auswertungseinheit sowie eine Bewegungskorrektureinheit aufweisen. Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen, welcher durch die Ansprüche gegeben ist.

## Patentansprüche

1. Verfahren zur Aufnahme von Magnetresonanzdaten eines Aufnahmebereichs eines Patienten, insbesondere wenigstens eines Teils eines Kopfes des Patienten, mit einer Magnetresonanzeinrichtung (35), wobei sich Aufnahmezeitabschnitte und Navigatorzeitabschnitte abwechseln, wobei in den Aufnahmezeitabschnitten Magnetresonanzdaten aufgenommen werden, und in den Navigatorzeitabschnitten (17) mittels einer Fettnavigatorsequenz, umfassend ein fettselektives Anregungsmodul mit wenigstens einem Hochfrequenzpuls und ein in-plane unterabtastendes Auslesemodul, Navigatordaten (20) aufgenommen werden, aus denen zur Bewegungskorrektur der Magnetresonanzdaten zu verwendende Bewegungsdaten ermittelt werden, **dadurch gekennzeichnet, dass** die Navigatordaten (20) mittels der simultane Mehrschichtbildgebung nutzenden Fettnavigatorsequenz nach dem auf mehrere aufzunehmende Fettnavigatorschichten wirkenden Anregungsmodul im Auslesemodul simultan aus den angeregten Fettnavigatorschichten aufgenommen werden, wobei wenigstens zeitweise in unterschiedlichen Navigatorzeitabschnitten (17) eine unterschiedliche Schichtgruppe (39, 25, 27, 29, 34) von Fettnavigatorschichten aufgenommen wird.

2. Verfahren nach Anspruch 1, **dadurch gekennzeichnet, dass** der in-plane-Unterabtastungsfaktor mehr als zwei, insbesondere mehr als zehn, beträgt und/oder wenigstens drei, bevorzugt wenigstens acht, Fettnavigatorschichten simultan aufgenommen werden.

3. Verfahren nach Anspruch 1 oder 2, **dadurch gekennzeichnet, dass** in dem Anregungsmodul eine Binomialpulsfolge (7, 8, 9) zur fettselektiven Anregung verwendet wird.

4. Verfahren nach Anspruch 3, **dadurch gekennzeichnet, dass** der Flipwinkel der Binomialpulsfolge (7, 8, 9) kleiner als 30°, insbesondere kleiner als 20°, gewählt wird und/oder dass Binomialpulsfolgen (7, 8, 9) für unterschiedliche Fettnavigatorschichten derart versetzt ausgegeben werden, dass wenigstens ein Hochfrequenzpuls (1, 2, 3, 11, 12, 13, 14, 15, 16) einer Binomialpulsfolge (7, 8, 9) zwischen zwei Hochfrequenzpulsen (1, 2, 3, 11, 12, 13, 14, 15, 16) der anderen Binomialpulsfolge (7, 8, 9) ausgegeben wird.

5. Verfahren nach einem der vorangehenden Ansprüche, **dadurch gekennzeichnet, dass** die Bewegungsdaten durch Vergleich der aktuell aufgenommenen Navigatordaten (20) mit einen vorherigen Bewegungszustand zeigenden Referenzdaten (26, 31) ermittelt werden, wobei die Navigatordaten (20) zu den Referenzdaten (26, 31) registriert werden.

6. Verfahren nach Anspruch 5, **dadurch gekennzeichnet, dass** die Referenzdaten (26, 31) Navigatordaten (20) wenigstens eines vorangehenden Navigatorzeitabschnitts (17) umfassen.

7. Verfahren nach Anspruch 6, **dadurch gekennzeichnet, dass** für wenigstens einen Navigatorzeitabschnitt (17), insbesondere wenigstens einen zeitlich ersten Navigatorzeitabschnitt (17), eine größere Zahl von Fettnavigatorschichten zur Erzeugung der Referenzdaten (26) aufgenommen werden und/oder die Referenzdaten 831) aus mehreren zeitlich aufeinanderfolgenden Navigatorzeitabschnitten (17), in denen unterschiedliche Fettnavigatorschichten simultan aufgenommen werden, zusammengestellt werden.

8. Verfahren nach einem der vorangehenden Ansprüche, **dadurch gekennzeichnet, dass** jeweils abwechselnd die unterschiedlichen Schichtgruppen (39, 25, 27, 29, 34) von Fettnavigatorschichten aufgenommen werden, wobei bei zwei abwechselnd aufgenommenen Schichtgruppen (27, 29, 34) von Fettnavigatorschichten diese ineinander verschachtelt sind.

9. Verfahren nach einem der vorangehenden Ansprüche, **dadurch gekennzeichnet, dass** die Schichtorientierung der Fettnavigatorschichten wenigstens einer der Schichtgruppen (27, 34) gegen die Schichtorientierung der Fettnavigatorschichten wenigstens einer anderen der Schichtgruppen (34, 27) gedreht ist, insbesondere um 90°.

10. Verfahren nach einem der vorangehenden Ansprüche, **dadurch gekennzeichnet, dass** die Bewegungsdaten für eine retrospektive oder eine prospektive Bewegungskorrektur genutzt werden.

11. Verfahren nach einem der vorangehenden Ansprüche, **dadurch gekennzeichnet, dass** die Magnetresonanzdaten mit einer Fettsignale unterdrückenden Magnetresonanzsequenz, insbesondere einer EPI-Sequenz, aufgenommen werden.

12. Verfahren nach einem der vorangehenden Ansprüche, **dadurch gekennzeichnet, dass** die Fettnavigatorsequenz eine EPI-Sequenz ohne Refokussierungspulse ist.

13. Magnetresonanzeinrichtung (35), aufweisend eine zur Durchführung eines Verfahrens nach einem der vorangehenden Ansprüche ausgebildete Steuereinrichtung (38).

14. Computerprogramm, welches die Schritte eines Verfahrens nach einem der Ansprüche 1 bis 12 durchführt, wenn es auf einer Steuereinrichtung (38) einer Magnetresonanzeinrichtung (35) ausgeführt wird.

15. Elektronisch lesbarer Datenträger, auf dem ein Computerprogramm nach Anspruch 14 gespeichert ist.

## Claims

1. Method for acquiring magnetic resonance data of an acquisition region of a patient, in particular at least a part of a head of the patient, by means of a magnetic resonance device (35), wherein acquisition time windows and navigator time windows alternate, wherein magnetic resonance data is acquired in the acquisition time windows, and navigator data (20) is acquired in navigator time windows (17) by means of a fat navigator sequence, comprising a fat-selective excitation module having at least one radiofrequency pulse and an in-plane undersampling readout module, from which navigator data (20) motion data that is to be used for the motion correction of the magnetic resonance data is determined,
**characterised in that** the navigator data (20) is acquired simultaneously from the excited fat navigator slices by means of the fat navigator sequence using simultaneous multislice imaging after the excitation module acting on a plurality of fat navigator slices to be acquired in the readout module, wherein a different group of slices (39, 25, 27, 29, 34) of fat navigator slices is acquired at least temporarily in different navigator time windows (17).

2. Method according to claim 1, **characterised in that** the in-plane undersampling factor amounts to more than two, in particular more than ten, and/or at least three, preferably at least eight, fat navigator slices are acquired simultaneously.

3. Method according to claim 1 or 2, **characterised in that** a binomial pulse sequence (7, 8, 9) is used for fat-selective excitation in the excitation module.

4. Method according to claim 3, **characterised in that** the flip angle of the binomial pulse sequence (7, 8, 9) is chosen as less than 30°, in particular less than 20°, and/or that binomial pulse sequences (7, 8, 9) for different fat navigator slices are output offset in such a way that at least one radiofrequency pulse (1, 2, 3, 11, 12, 13, 14, 15, 16) of a binomial pulse sequence (7, 8, 9) is output between two radiofrequency pulses (1, 2, 3, 11, 12, 13, 14, 15, 16) of the other binomial pulse sequence (7, 8, 9).

5. Method according to one of the preceding claims, **characterised in that** the motion data is determined by comparison of the currently acquired navigator data (20) with reference data (26, 31) showing a previous motion state, wherein the navigator data (20) is registered with the reference data (26, 31).

6. Method according to claim 5, **characterised in that** the reference data (26, 31) comprises navigator data (20) of at least one preceding navigator time window (17).

7. Method according to claim 6, **characterised in that** for at least one navigator time window (17), in particular at least one first navigator time window (17) in time sequence, a greater number of fat navigator slices are acquired for generating the reference data (26) and/or the reference data (31) is compiled from a plurality of navigator time windows (17) succeeding one another in time, in which different fat navigator slices are acquired simultaneously.

8. Method according to one of the preceding claims, **characterised in that** the different slice groups (39, 25, 27, 29, 34) of fat navigator slices are acquired alternately in each case, wherein with two alternately acquired slice groups (27, 29, 34) of fat navigator slices these are interleaved in one another.

9. Method according to one of the preceding claims, **characterised in that** the slice orientation of the fat navigator slices of at least one of the slice groups (27, 34) is rotated against the slice orientation of the fat navigator slices of at least one other of the slice groups (34, 27), in particular by 90°.

10. Method according to one of the preceding claims, **characterised in that** the motion data is used for a retrospective or a prospective motion correction.

11. Method according to one of the preceding claims, **characterised in that** the magnetic resonance data is acquired by means of a magnetic resonance sequence suppressing fat signal, in particular an EPI sequence.

12. Method according to one of the preceding claims, **characterised in that** the fat navigator sequence is an EPI sequence without refocusing pulses.

13. Magnetic resonance device (35) having a control device (38) embodied for carrying out a method according to one of the preceding claims.

14. Computer program which performs the steps of a method according to one of claims 1 to 12 when it is executed on a control device (38) of a magnetic resonance device (35).

15. Electronically readable data medium on which a computer program according to claim 14 is stored.

## Revendications

1. Procédé d'enregistrement de données de résonnance magnétique d'une partie à enregistrer d'un patient, notamment d'au moins une partie de la tête du patient, comprenant un dispositif (35) de résonnance magnétique, dans lequel des intervalles de temps d'enregistrement et des intervalles de temps de navigateur alternent, dans lequel on enregistre des données de résonnance magnétique dans les intervalles de temps d'enregistrement et on enregistre, dans les intervalles (17) de temps de navigateur au moyen d'une séquence de navigateur pour la graisse, comprenant un module d'excitation sélectif pour la graisse ayant au moins une impulsion de haute fréquence et un module de lecture sous-échantillonné in-plane, des données (20) de navigateur, à partir desquelles on détermine des données de déplacement à utiliser pour la correction de déplacement des données de résonnance magnétique, **caractérisé en ce que** l'on enregistre les données (20) de navigateur, au moyen de la séquence de navigateur pour la graisse utilisant l'imagerie multi-tranches simultanée suivant le module d'excitation agissant sur plusieurs tranches de navigateur pour la graisse enregistrées, dans le module de lecture simultanément à partir des tranches de navigateur pour la graisse excitées, dans lequel au moins de temps à autre, dans des intervalles (17) de temps de navigateur différents, on enregistre un groupe (39, 25, 27, 29, 34) différents de tranches de navigateur pour la graisse.

2. Procédé suivant la revendication 1, **caractérisé en ce que** le facteur de sous-échantillonnage in-plane est de plus de deux, notamment de plus de dix et/ou on enregistre simultanément au moins trois, de préférence au moins huit, tranches de navigateur pour la graisse.

3. Procédé suivant la revendication 1 ou 2, **caractérisé en ce que** l'on utilise, dans le module d'excitation, une séquence (7, 8, 9) d'impulsions binomiale pour l'excitation sélective pour la graisse.

4. Procédé suivant la revendication 3, **caractérisé en ce que** l'on choisit l'angle de flip de la séquence (7 , 8, 9) d'impulsions binomiale plus petit que 30°, notamment plus petit que 20° et/ou **en ce que** l'on émet, de manière décalée, des séquences (7, 8, 9) d'impulsions binomiales pour des tranches de navigateur pour la graisse différentes, de manière à émettre au moins une impulsion (1, 2, 3, 11, 12, 13, 14, 15, 16) de haute fréquence d'une séquence (7, 8, 9) d'impulsions binomiale entre deux impulsions (1, 2, 3, 11, 12, 13 , 14, 15, 16) de haute fréquence des autres séquences (7, 8, 9) d'impulsions binomiales.

5. Procédé suivant l'une des revendications précédentes, **caractérisé en ce que** l'on détermine les données de déplacement en comparant les données (20) de navigateur enregistrées en cours à des données (26, 31) de référence indiquant un état de déplacement précédent, les données (20) de navigateur étant en correspondance avec les données (26, 31) de référence.

6. Procédé suivant la revendication 5, **caractérisé en ce que** les données (26, 31) de référence comprennent des données (20) de navigateur d'au moins un intervalle (17) de temps de navigateur précédent.

7. Procédé suivant la revendication 6, **caractérisé en ce que**, pour au moins un intervalle (17) de temps de navigateur, notamment pour au moins un premier dans le temps intervalle (17) de temps de navigateur, on enregistre un nombre plus grand de tranches de navigateur pour la graisse, pour la production des données (26) de référence et/ou on combine des données (31) de référence de plusieurs intervalles (17) de temps de navigateur se succédant dans le temps, dans lequel on enregistre simultanément des tranches différentes de navigateur pour la graisse.

8. Procédé suivant l'une des revendications précédentes, **caractérisé en ce que** l'on enregistre respectivement en alternance les groupes (39, 25, 27, 29, 34) différents de tranches de navigateur pour la graisse, dans lequel, pour deux groupes (27, 29, 34) de tranches enregistrés en alternance de tranches de navigateur pour la graisse, on les imbrique l'un dans l'autre.

9. Procédé suivant l'une des revendications précédentes, **caractérisé en ce que** l'orientation des tranches de navigateur pour la graisse d'au moins l'un des groupes (27, 34) de tranches est tournée, par rapport à l'orientation des tranches de navigateur pour la graisse, d'au moins un autre des groupes (34, 27) de tranches, notamment de 90°.

10. Procédé suivant l'une des revendications précédentes, **caractérisé en ce que** l'on utilise les données de déplacement, pour une correction de déplacement rétrospective ou prospective.

11. Procédé suivant l'une des revendications précédentes, **caractérisé en ce que** l'on enregistre les données de résonnance magnétique par une séquence de résonnance magnétique supprimant des signaux pour la graisse, notamment par une séquence EPI.

12. Procédé suivant l'une des revendications précédentes, **caractérisé en ce que** la séquence de navigateur pour la graisse est une séquence EPI sans impulsion de refocalisation.

13. Dispositif (35) de résonnance magnétique comportant un dispositif (38) de commande, constitué pour effectuer un procédé suivant l'une des revendications précédentes.

14. Programme d'ordinateur, qui effectue les stades d'un procédé suivant l'une des revendications 1 à 12, lorsqu'il est réalisé sur un dispositif (38) de commande d'un dispositif (35) de résonnance magnétique.

15. Support de données, déchiffrables électroniquement, sur lequel est mis en mémoire un programme d'ordinateur suivant la revendication 14.
